(19) Europäisches Patentamt
European Patent Office
Office européen des brevets

(11) Veröffentlichungsnummer: **0 268 964 B1**

# (12) EUROPÄISCHE PATENTSCHRIFT

(45) Veröffentlichungstag der Patentschrift: **15.05.91**

(51) Int. Cl.5: **C07D 213/803**, C07D 213/80, C07C 229/00, C07C 227/00

(21) Anmeldenummer: **87116841.5**

(22) Anmeldetag: **14.11.87**

## (54) Verfahren zur Herstellung von 6-Hydroxy-3-pyridincarbonsäureestern.

(30) Priorität: **27.11.86 DE 3640583**

(43) Veröffentlichungstag der Anmeldung:
**01.06.88 Patentblatt 88/22**

(45) Bekanntmachung des Hinweises auf die Patenterteilung:
**15.05.91 Patentblatt 91/20**

(84) Benannte Vertragsstaaten:
**BE CH DE FR GB IT LI NL**

(56) Entgegenhaltungen:
**GB-A- 867 930**

**CHEMICAL ABSTRACTS, Band 81, Nr. 13, 30.September 1974, Colombus,Ohio,USA N.ANGHELIDE et al. "New dienamino esters and their cyclisation to alpha pyridones of nicotinic acid" Seite 420, Spalte 2, Zusammenfassung Nr. 77 429b**

(73) Patentinhaber: **BAYER AG**

**W-5090 Leverkusen 1 Bayerwerk(DE)**

(72) Erfinder: **Maurer, Fritz, Dr.**
**Roeberstrasse 8**
**W-5600 Wuppertal(DE)**

EP 0 268 964 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von 6-Hydroxy-3-pyridincarbonsäureestern, Zwischenprodukte welche für die Durchführung des Verfahrens verwendet werden können, sowie Verfahren zur Herstellung solcher Zwischenprodukte. Die Verfahrensprodukte sind bekannt und können als Zwischenprodukte zur Herstellung von Insektiziden verwendet werden.

Es ist bereits bekannt, daß 6-Hydroxy-3-pyridincarbonsäureester erhalten werden können, wenn man Hydroxybernsteinsäureester mit rauchender Schwefelsäure erhitzt und die gebildeten Cumalinsäure-Derivate mit Ammoniak umsetzt. Nachteilig bei dieser Reaktion ist der Einsatz von größeren Mengen rauchender Schwefelsäure, die bei der Aufarbeitung wieder aus dem Reaktionsgemisch entfernt werden muß und ökologisch nicht einfach zu handhaben ist (vgl. Ber. 17, 2384 (1884), Helv. Chim. Acta 4, 482 (1921) und J. Am. chem. Soc. 66, 1482 (1944)).

Weiterhin ist bekannt, daß die 6-Hydroxy-3-pyridincarbonsäureester bzw. die entsprechenden tautomeren α-Pyridoncarbonsäureester hergestellt werden können, wenn man Enamin-Derivate mit aprotischen polaren Verdünnungsmitteln bei Temperaturen zwischen 160 °C - 190 °C umsetzt. Der Nachteil bei diesem Verfahren ist die relativ hohe Reaktionstemperatur und der dadurch bedingte hohe Energieaufwand (vgl. Tetrahedron, S. 623 - 632, (1974)).

Es wurde nun gefunden, daß man 6-Hydroxy-3-pyridincarbonsäureester der allgemeinen Formel (I)

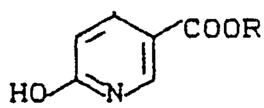

(I)

in welcher
R für $C_1$-$C_6$-Alkyl steht,
erhält, wenn man
1-Dialkylamino-1,3-butadien-2,4-dicarbonsäureester der Formel (II)

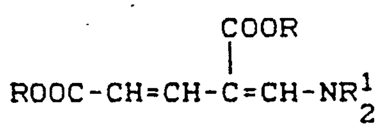

(II)

in welcher
R die oben angegebene Bedeutung hat und
$R^1$ für $C_1$-$C_6$-Alkyl steht,
entweder

(a) mit gasförmigem Ammoniak, in Gegenwart von Alkalialkoholaten und in Gegenwart von aromatischen Kohlenwasserstoffen als Verdünnungsmittel bei Temperaturen zwischen 0 °C und 120 °C umsetzt, wobei auf 1 Mol der Verbindung (II) 1 bis 1,5 Mol Alkalialkoholat und 2 bis 10 Mol gasförmiges Ammoniak eingesetzt werden,

oder

(b) in einer Zweistufenreaktion mit gasformigem Ammoniak und in Gegenwart von Verdünnungsmitteln bei Temperaturen zwischen 0 °C und 50 °C zu den Amino-Derivaten der Formel (III)

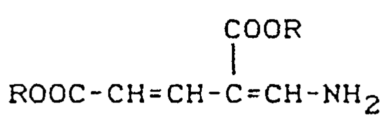

(III)

in welcher
R die oben angegebene Bedeutung hat,

umsetzt, wobei auf 1 Mol der Verbindung (II) 2 bis 10 Mol gasförmiges Ammoniak eingesetzt werden, die dabei erhaltenen Verbindungen der Formel (III) gegebenenfalls isoliert und anschließend die Amino-Derivate

der Formel (III) in einer 2.Reaktionsstufe mit Alkalialkoholaten und in Gegenwart von niedrigen Alkoholen als Verdünnungsmittel bei Temperaturen zwischen 0° und 120°C umsetzt, wobei auf 1 Mol der Verbindung (III) 1 bis 1,5 Mol Alkalialkoholat eingesetzt werden.

Überraschenderweise lassen sich mit Hilfe des erfindungsgemäßen Verfahrens (Variante (a) und (b)) auf einfache Weise und ausgehend von preisgünstigen neuen Zwischenprodukten, die Verbindungen der Formel (I) in guten Ausbeuten herstellen.

Bevorzugt werden mit Hilfe des erfindungsgemäßen Verfahrens die Verbindungen der Formel (I) hergestellt, in welcher
R für $C_1$-$C_4$-Alkyl steht.

Die Herstellung der Verbindungen der Formel (I) wird bevorzugt gemäß der Verfahrensvariante (a) ("Eintopf-Verfahren") durchgeführt.

Verwendet man für das erfindungsgemäße Verfahren gemäß Variante (a) 1-Dimethylamino-1,3-butadien-2,4-dicarbonsäurediethylester, Ammoniak und Natriumethylat als Ausgangsstoffe, so kann die Reaktion durch das folgende Formelschema skizziert werden:

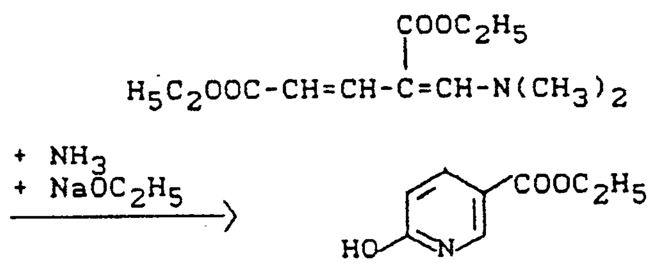


Verwendet man für das erfindungsgemäße Verfahren gemäß Variante (b) 1-Dimethylamino-1,3-butadien-2,4-dicarbonsäurediethylester und Ammoniak als Ausgangsstoffe, isoliert den gebildeten 1-Amino-1,3-butadien-2,4-dicarbonsäurediethylester und setzt diesen Ester weiter um mit Natriumethylat, so kann die Reaktionsfolge durch das folgende Formelschema skizziert werden:

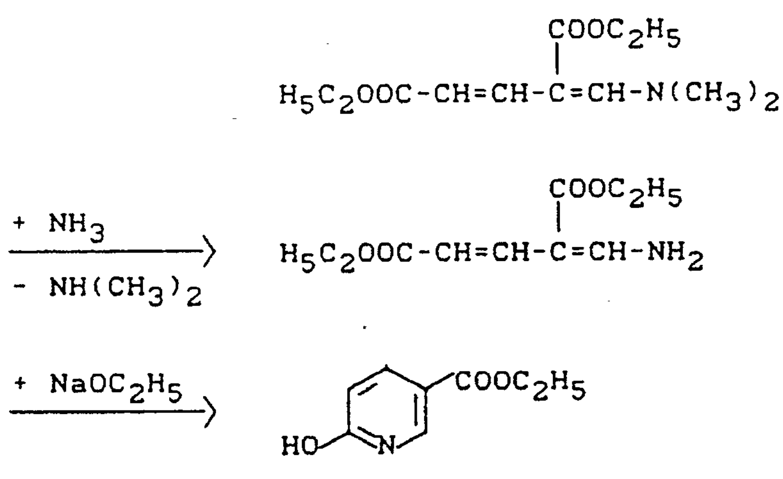


Die als Ausgangsstoffe für das erfinderische Verfahren (Variante (a) und (b)) zu verwendenden 1-Dialkylamino-1,3-butadien-2,4-dicarbonsäureester sind durch die Formel (II) allgemein definiert. In dieser Formel steht R bevorzugt für diejenigen Reste, welche oben im Rahmen der Substituentendefinition der Formel (I) vorzugsweise bzw. als insbesondere bevorzugt angegeben sind. $R^1$ steht in dieser Formel vorzugsweise für $C_1$-$C_4$-Alkyl.

Die Verbindungen der Formel (II) sind neu und Teil der vorliegenden Erfindung. Sie lassen sich auf einfache Weise herstellen, indem man z. B. Glutaconsäureester der Formel (IV)

$ROOC\text{-}CH = CH_2COOR$   (IV)

in welcher
R die oben angegebene Bedeutung hat,
mit N,N-Dialkylformamidacetalen der Formel (V)

$(R^1)_2N\text{-}CH(OR^2)_2$ (V)

in welcher
$R^1$ die oben angegebene Bedeutung hat und
$R^2$ für $C_1$-$C_6$-Alkyl steht,
gegebenenfalls in Gegenwart von Säureanhydriden wie z. B. Essigsäureanhydrid und gegebenenfalls in Gegenwart von inerten Verdünnungsmitteln wie z. B. Toluol bei Temperaturen zwischen 0 °C und 40 °C umsetzt.

Die als Ausgangsverbindungen zu verwendenden Glutaconsäureester sind durch die Formel (IV) allgemein definiert. In dieser Formel steht R vorzugsweise für diejenigen Reste, welche oben im Rahmen der Substitutendefinition der Formel (I) vorzugsweise bzw. als insbesondere bevorzugt angegeben sind.

Die Glutaconsäureester der Formel (IV) sind bekannte Verbindungen der organischen Chemie.

Als Beispiele für die Verbindungen der Formel (IV) seien genannt:
Glutaconsäure-dimethylester, -diethylester, -di-n-propylester, di-i-propylester und -di-n-butylester.

Die weiterhin als Ausgangsstoffe zur Herstellung der neuen Verbindungen der Formel (II) zu verwendenden N,N-Dialkylformamidacetale sind durch die Formel (V) allgemein definiert. In dieser Formel stehen $R^1$ und $R^2$ bevorzugt für $C_1$-$C_4$-Alkyl.

Die N,N-Dialkylformamidacetale der Formel (V) sind allgemein bekannte Verbindungen der organischen Chemie.

Als Beispiele für die Verbindungen der Formel (V) seien genannt :
N,N-Dimethylformamiddimethylacetal, N,N-Dimethylformamiddiethylacetal, N,N-Dimethylformamiddi-n-propylacetal, N,N-Dimethylformamiddi-i-propylacetal und N,N-Dimethylformamiddi-n-butylacetal. Bevorzugt wird N,N-Dimethylformamiddimethylacetal als Reaktionskomponente zur Herstellung der neuen Verbindungen eingesetzt.

Die als Ausgangsstoffe für das erfinderische Verfahren (Variante (b)/2. Stufe) zu verwendenden Amino-Derivate sind durch die Formel (III) allgemein definiert. In dieser Formel steht R bevorzugt für diejenigen Reste, welche oben im Rahmen der Substituentendefinition der Formel (I) vorzugsweise bzw. als insbesondere bevorzugt angegeben sind.

Die Verbindungen der Formel (III) sind neu und Teil der vorliegenden Erfindung. Sie lassen sich auf einfache Weise nach dem erfinderischen Verfahren (Variante (b), 1. Stufe) herstellen.

Als Beispiele für die Amino-Derivate der Formel (III) seien genannt:
1-Amino-1,3-butadien-2,4-dicarbonsäure-dimethylester, -diethylester, -di-n-propylester, -di-i-propylester und -di-butylester.

Als Verdünnungsmittel kommen dabei für die Variante (a) vorzugsweise Xylol, Toluol und Benzol in Frage.
Für die Verfahrensvariante (b) kommen für die 1. und 2. Stufe als Verdünnungsmittel Methanol, Ethanol, n-Propanol, i-Propanol, n-Butanol, i-Butanol, sec.-Butanol und tert.-Butanol in Frage.

Als Alkalialkoholate kommen für die Verfahrensvarianten (a) und ((b)/Stufe 2) vorzugsweise Natrium- und Kaliummethylat und -ethylat sowie Kalium-tert.-butylat in Frage. Besonders bevorzugt werden Natriummethylat und -ethylat eingesetzt.

Die Reaktionstemperaturen können bei dem erfindungsgemäßen Verfahren (Variante (a) und (b)) in einem größeren Bereich variiert werden. Man arbeitet bei den Verfahrensvarianten (a) und ((b/Stufe 2) bei Temperaturen zwischen 0 °C und 120 °C, vorzugsweise bei Temperaturen zwischen 20 °C und 100 °C; und bei der Verfahrensvariante ((b)/Stufe 1) bei Temperaturen zwischen 0 °C und 50 °C, vorzugsweise bei Temperaturen zwischen 10 °C und 35 °C. Das erfindungsgemäße Verfahren wird vorzugsweise unter Normaldruck durchgeführt.

Zur Durchführung des erfindungsgemäßen Verfahrens (Variante (a)) setzt man auf 1 Mol der Verbindung der Formel (II), 1 bis 1,5 Mol, vorzugsweise 1 bis 1,3 Mol Alkalialkoholat und 2 bis 10 Mol, vorzugsweise 3 bis 6 Mol gasförmigen Ammoniak ein. Man geht im allgemeinen so vor, daß zu einer Lösung aus Alkoholat und Alkohol bis zur Sättigung Ammoniak eingeleitet wird. Anschließend versetzt man die Lösung mit der Verbindung der Formel (II) und rührt mehrere Stunden bei der erforderlichen Temperatur. Zur Freisetzung der Verbindung der Formel (I) wird konzentrierte Salzsäure in der Weise zugegeben, daß ein pH-Wert = 4 erreicht wird. Die weitere Aufarbeitung geschieht nach üblichen Methoden.

Zur Durchführung des erfindungsgemäßen Verfahrens (Variante (b)) setzt man in der 1. Stufe auf 1 Mol der Verbindung der Formel (II) 2 bis 10 Mol, vorzugsweise 3 bis 6 mol gasförmigen Ammoniak ein. Man geht im allgemeinen so vor, daß Ammoniak in Alkohol eingeleitet wird und zu diesem Gemisch die erforderliche Menge der Verbindung der Formel (II) zugegeben wird und man mehrere Stunden bei der erforderlichen Temperatur rührt. Nach gegebenenfalls erfolgter Aufarbeitung und Isolierung der Verbindung der Formel (III) geht man in der 2, Stufe so vor, daß 1 Mol der Verbindung der Formel (III) in Gegenwart von Alkohol und 1 bis 1,5 Mol, vorzugsweise 1 bis 1,3 Mol Alkoholat mehrere Stunden bei der erforderlichen Temperatur gerührt wird.

Zur Freisetzung der Verbindung der Formel (I) wird konzentrierte Salzsäure in der Weise zugegeben, daß ein pH-Wert = 4 erreicht wird. Die Aufarbeitung geschieht nach üblichen Methoden.

Die nach dem erfinderischen Verfahren herzustellenden 6-Hydroxy-3-pyridincarbonsäureester der Formel (I) können als Zwischenprodukte für die Herstellung von Nitromethylen-Derivaten, die als Insektizide wirksam sind, eingesetzt werden (vgl. EP-A 163 855 und EP-A 192 060).

Die Weiterverarbeitung der Verbindung der Formel (I) zu bekannten Insektiziden sei beispielhaft anhand des folgenden Schemas verdeutlicht:

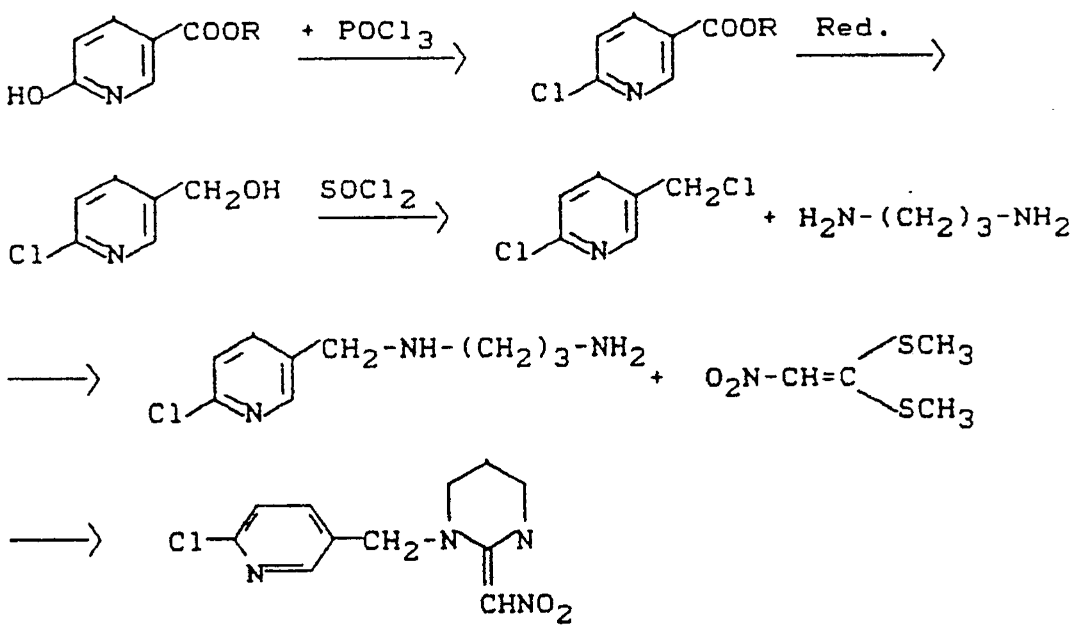


Herstellungsbeispiele

Beispiel 1

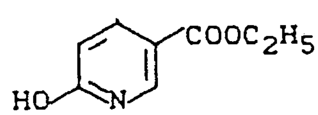

(Verfahrensvariante (a))

Zu einer Lösung von 2,1 g (0,12 Mol) Ammoniakgas und 2,04 g (0,03 Mol) Natriumethylat in 50 ml Ethanol gibt man bei 5 °C 6 g (0,025 Mol) 1-Dimethylamino-1,3-butadien-2,4-dicarbonsäurediethylester und kocht das Gemisch 18 Stunden unter Rückfluß. Dann destilliert man das Lösungsmittel im Vakuum ab, löst den Rückstand in 60 ml Wasser, stellt durch Zugabe von konzentrierter Salzsäure auf pH 4 ein und

extrahiert 3 mal mit je 50 ml Methylenchlorid. Die organischen Phasen werden über Natriumsulfat getrocknet und im Vakuum eingedampft.

Man erhält so 3,6 g (86 % der Theorie) 6-Hydroxy-3-pyridincarbonsäureethylester in Form beiger Kristalle vom Schmelzpunkt 143 °C - 144 °C.

(Verfahrensvariante (b))

Eine Mischung aus 0,75 g (0,011 Mol) Natriumethylat, 15 ml Methanol und 2,1 g (0,01 Mol) 1-Amino-1,3-butadien-2,4-dicarbonsäurediethylester wird 18 Stunden unter Rückfluß gekocht. Dann destilliert man das Lösungsmittel im Vakuum ab, löst den Rückstand in 20 ml Wasser und stellt durch Zugabe von Salzsäure auf pH 4 ein. Man extrahiert 3 mal mit je 100 ml Methylenchlorid, trocknet die organische Phase über Natriumsulfat und destilliert das Lösungsmittel im Vakuum ab.

Man erhält 1,4 g (84 % der Theorie) 6-Hydroxy-3-pyridincarbonsäureethylester von Schmelzpunkt 144 °C.

Analog Beispiel 1 bzw. den beiden angegebenen Verfahrensvarianten (a) und (b) können die folgenden Verbindungen der Formel (I) hergestellt werden:

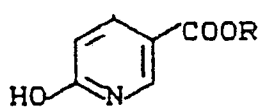

(I)

Beispiel 2

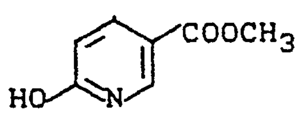

Fp: 147 °C

Beispiel 3

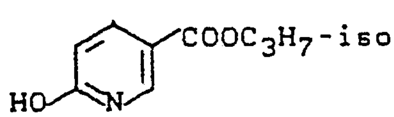

Ausgangsverbindungen der Formel (II)

Beispiel (II-1)

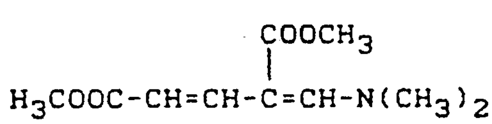

Zu einer Mischung aus 28 g (0,18 Mol) Glutaconsäuredimethylester, 36,7 g (0,36 Mol) Essigsäureanhydrid und 200 ml Toluol tropft man 25,4 g (0,21 Mol) Dimethylformamiddimethylacetal. Das Reaktionsgemisch erwärmt sich dabei auf ca. 27 °C; es wird 18 Stunden ohne Kühlung gerührt und dann bei 50 °C im Vakuum eingedampft. Den Rückstand befreit man bei 80 °C im Hochvakuum von flüchtigen Anteilen.

Man erhält 30 g (78 % der Theorie) 1-Dimethylamino-1,3-butadien-2,4-dicarbonsäuredimethylester in Form beiger Kristalle vom Schmelzpunkt 62 °C.

Analog Beipsiel (II-1) können die folgenden Verbindungen der Formel (II) hergestellt werden:

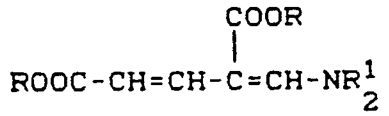

(II)

Beispiel (II-2)

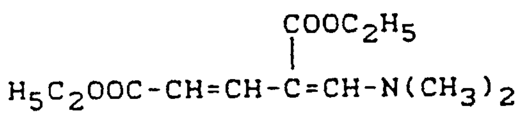

$n_D^{23}$ : 1,5715

Beispiel (II-3)

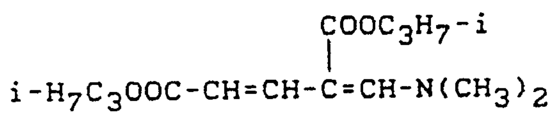

Beispiel (II-4)

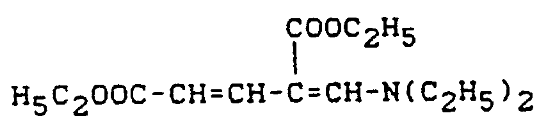

Ausgangsverbindungen der Formel (III)

Beispiel 1

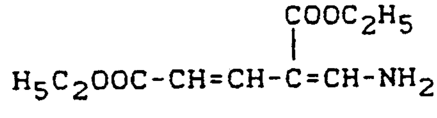

2,1 g (0,125 Mol) Ammoniakgas werden in 50 ml Ethanol gelöst. Zu dieser Mischung gibt man 6 g (0,025 Mol) 1-Dimethylamino-1,3-butadien-2,4-dicarbonsäurediethylester und kocht das Reaktionsgemisch 24 Stunden unter Rückfluß. Dann destilliert man das Lösungsmittel im Vakuum ab, verreibt den Rückstand mit Petrolethyer und saugt das kristalline Produkt ab.

Man erhält 4,5 g (84 % der Theorie) 1-Amino-1,3-butadien-2,4-dicarbonsäurediethylester in Form beiger Kristalle vom Schmelzpunkt 109 °C.

Analog Beispiel (III-1) können die folgenden Verbindungen der Formel (III) hergestellt werden:

$$\begin{array}{l} \qquad\qquad\qquad\;\; COOR \\ \qquad\qquad\qquad\quad\;\; | \\ ROOC-CH=CH-C=CH-NH_2 \end{array} \qquad (III)$$

Beispiel (III-2)

$$\begin{array}{l} \qquad\qquad\qquad\qquad\; COOCH_3 \\ \qquad\qquad\qquad\qquad\;\; | \\ H_3COOC-CH=CH-C=CH-NH_2 \end{array} \qquad (III) \qquad Fp:\ 151\ ^\circ C$$

Beispiel (III-3)

$$\begin{array}{l} \qquad\qquad\qquad\qquad\qquad\quad COOC_3H_7-i \\ \qquad\qquad\qquad\qquad\qquad\quad\; | \\ i-H_7C_3OOC-CH=CH-C=CH-NH_2 \end{array}$$

## Ansprüche

1. Verfahren zur Herstellung von 6-Hydroxy-3-pyridin-carbonsäureestern der allgemeinen Formel (I)

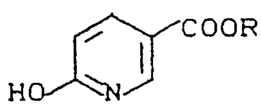

(I)

in welcher

R für $C_1$-$C_6$-Alkyl steht,

dadurch gekennzeichnet, daß man

1-Dialkylamino-1,3-butadien-2,4-dicarbonsäureester der Formel (II)

$$\begin{array}{l} \qquad\qquad\qquad\;\; COOR \\ \qquad\qquad\qquad\quad\;\; | \\ ROOC-CH=CH-C=CH-NR^1_2 \end{array} \qquad (II)$$

in welcher

R die oben angegebene Bedeutung hat und

$R^1$ für $C_1$-$C_6$-Alkyl steht,

entweder

(a) mit gasförmigem Ammoniak, in Gegenwart von Alkalialkoholaten und in Gegenwart von aromatischen Kohlenwasserstoffen als Verdünnungsmittel bei Temperaturen zwischen 0 °C und 120 °C umsetzt, wobei auf 1 Mol der Verbindung (II) 1 bis 1,5 Mol Alkalialkoholat und 2 bis 10 Mol gasförmiges Ammoniak eingesetzt werden,

oder

(b) in einer Zweistufenreaktion mit gasförmigem Ammoniak und in Gegenwart von Verdünnungsmitteln bei Temperaturen zwischen 0 °C und 50 °C zu den Amino-Derivaten der Formel (III)

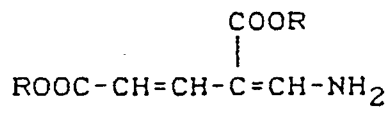 (III)

in welcher
R die oben angegebene Bedeutung hat,
umsetzt, wobei auf 1 Mol der Verbindung (II) 2 bis 10 Mol gasförmiges Ammoniak eingesetzt werden, die dabei erhaltenen Verbindungen der Formel (III) gegebenenfalls isoliert und anschließend die Amino-Derivate der Formel (III) in einer 2.Reaktionsstufe mit Alkalialkoholaten und in Gegenwart von niedrigen Alkoholen als Verdünnungsmittel bei Temperaturen zwischen 0° und 120° C umsetzt, wobei auf 1 Mol der Verbindung (III) 1 bis 1,5 Mol Alkalialkoholat eingesetzt werden.

**2.** Verfahren gemäß Anspruch 1 dadurch gekennzeichnet, daß man bei Variante (a) bei 20 bis 100° C, in der
1.Stufe der Variante (b) bei 10 bis 35 °C und in der
2.Stufe der Variante (b) bei 20 bis 100 °C arbeitet.

**3.** Verbindungen der allgemeinen Formel

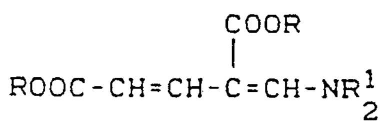 (II)

in welcher
R für $C_1$-$C_6$-Alkyl steht
und
$R^1$ für $C_1$-$C_6$-Alkyl steht.

**4.** Verfahren zur Herstellung von Verbindungen der Formel

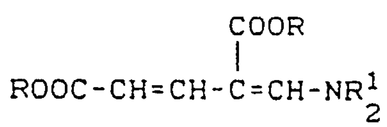 (II)

in welcher
R für $C_1$-$C_6$-Alkyl steht und
$R^1$ für $C_1$-$C_6$-Alkyl steht,
dadurch gekennzeichnet, daß man Glutaconsäureester der Formel (IV)

$ROOC\text{-}CH=CH\text{-}CH_2\text{-}COOR$ (IV)

in welcher
R die oben angegebene Bedeutung hat, mit N,N-Dialkylformamidacetalen der Formel (V)

$(R^1)_2N\text{-}CH(OR^2)_2$ (V)

in welcher
$R^1$ die oben angegebene Bedeutung hat und
$R^2$ für $C_1$-$C_6$-Alkyl steht,
gegebenenfalls in Gegenwart von Säureanhydriden und gegebenenfalls in Gegenwart von inerten Verdünnungsmitteln zwischen 0 °C und 40 °C umsetzt.

**5.** Verbindungen der Formel (III)

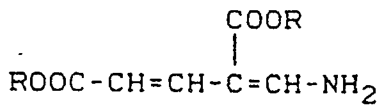 (III)

in welcher
R für $C_1$-$C_6$-Alkyl steht,

**6.** Verfahren zur Herstellung von Verbindungen der Formel (III)

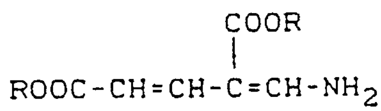 (III)

in welcher
R für $C_1$-$C_6$-Alkyl steht.
dadurch gekennzeichnet, daß man
1-Dialkylamino-1,3-butadien-2,4-dicarbonsäureester der Formel (II)

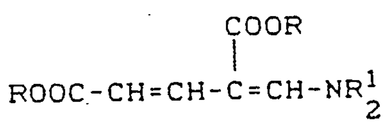 (II)

in welcher
R die oben angegebene Bedeutung hat und
$R^1$ für $C_1$-$C_6$-Alkyl steht,
mit gasförmigem Ammoniak und in Gegenwart von Verdünnungsmitteln bei Temperaturen zwischen 0° C und 50° C umsetzt.

## Claims

**1.** Process for the preparation of 6-hydroxy-3-pyridinecarboxylic acid esters of the general formula (I)

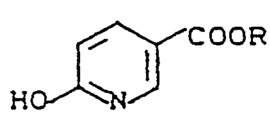 (I)

in which
R represents $C_1$-$C_6$-alkyl,
characterised in that 1-dialkylamino-1,3-butadiene-2,4-dicarboxylic acid esters of the formula (II)

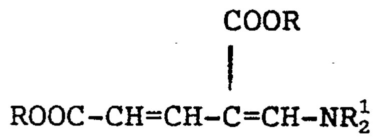

(II)

in which

R has the abovementioned meaning and

$R^1$ represents $C_1$-$C_6$-alkyl,

either

(a) are reacted with gaseous ammonia in the presence of alkali metal alcoholates and in the presence of aromatic hydrocarbons as diluents at temperatures between 0° C and 120° C wherein 1 to 1.5 mol of alkali metal alcoholate and 2 to 10 mol of gaseous ammonia are employed per mol of the compound (II),

or

(b) in a two-stage reaction, are reacted with gaseous ammonia and in the presence of diluents at temperatures between 0° C and 50° C to give the amino derivatives of the formula (III)

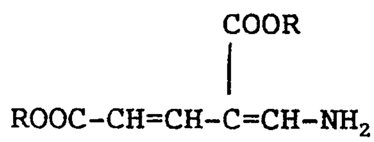

(III)

in which

R has the abovementioned meaning,

wherein 2 to 10 mol of gaseous ammonia are employed per mol of the compound (II), if appropriate the resulting compounds of the formula (III) are isolated, and the amino derivatives of the formula (III) are then reacted in a 2nd reaction stage with alkali metal alcoholates and in the presence of lower alcohols as diluents at temperatures between 0° C and 120° C, wherein 1 to 1.5 mol of alkali metal alcoholate are employed per mol of the compound (III).

**2.** Process according to Claim 1, characterised in that the reaction is carried out in variant (a) at 20 to 100° C, in the 1st stage of variant (b) at 10 to 35° C and in the 2nd stage of variant (b) at 20 to 100° C.

**3.** Compounds of the general formula

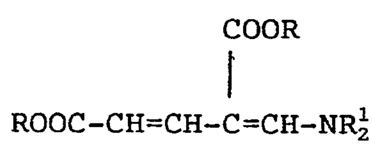

(II)

in which

R represents $C_1$-$C_6$-alkyl and

$R^1$ represents $C_1$-$C_6$-alkyl.

**4.** Process for the preparation of compounds of the formula

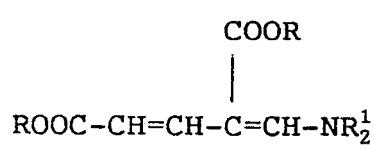

(II)

in which

R represents $C_1$-$C_6$-alkyl and
$R^1$ represents $C_1$-$C_6$-alkyl,
characterised in that glutaconic acid esters of the formula (IV)

$ROOC\text{-}CH=CH\text{-}CH_2\text{-}COOR$ (IV)

in which
R has the abovementioned meaning,
are reacted with N,N-dialkylformamide acetals of the formula (V)

$(R^1)_2N\text{-}CH(OR^2)_2$ (V)

in which
$R^1$ has the abovementioned meaning and
$R^2$ represents $C_1$-$C_6$-alkyl,
if appropriate in the presence of acid anhydrides and if appropriate in the presence of inert diluents at between 0°C and 40°C.

**5.** Compounds of the formula (III)

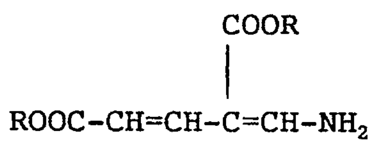

(III)

in which
R represents $C_1$-$C_6$-alkyl.

**6.** Process for the preparation of compounds of the formula (III)

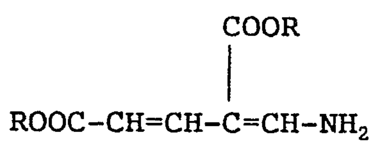

(III)

in which
R represents $C_1$-$C_6$-alkyl,
characterised in that 1-diakylamino-1,3-butadiene-2,4-dicarboxylic acid esters of the formula (II)

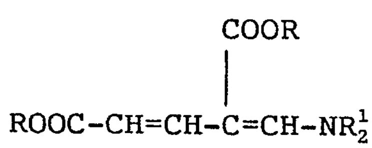

(II)

in which
R has the abovementioned meaning and
$R^1$ represents $C_1$-$C_6$-alkyl,
are reacted with gaseous ammonia and in the presence of diluents at temperatures between 0°C and 50°C.

**Revendications**

**1.** Procédé de préparation d'esters d'acide 6-hydroxy-3-pyridine-carboxylique de formule générale (I)

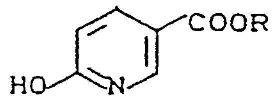

(I)

dans laquelle

R est un groupe alkyle en $C_1$ à $C_6$,

caractérisé en ce qu'on fait réagir des esters d'acides 1-dialkylamino-1,3-butadiène-2,4-dicarboxyliques de formule (II)

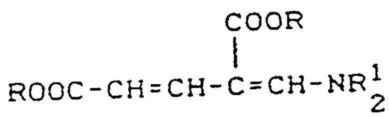

(II)

dans laquelle

R a la définition indiquée ci-dessus et

$R^1$ est un groupe alkyle en $C_1$ à $C_6$,

ou bien

(a) avec l'ammoniac gazeux, en présence d'alcoolates alcalins et en présence d'hydrocarbures aromatiques comme diluants à des températures comprises entre 0° C et 120° C, en utilisant par mole de composé (II) 1 à 1,5 mole d'alcoolate alcalin et 2 à 10 moles d'ammoniac gazeux, ou bien en les faisant réagir

(b) dans une réaction à deux étapes avec l'ammoniac gazeux et en présence de diluants à des températures comprises entre 0° C et 50° C pour former des dérivés aminés de formule (III)

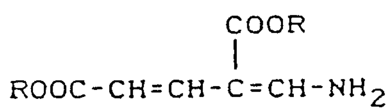

(III)

dans laquelle

R a la définition indiquée ci-dessus,

en utilisant par mole de composé (II) 2 à 10 moles d'ammoniac gazeux, en isolant éventuellement les composés obtenus de formule (III), puis en faisant réagir les dérivés aminés de formule (III) dans une seconde étape réactionnelle avec des alcoolates alcalins et en présence d'alcools inférieurs comme diluants à des températures comprises entre 0 et 120° C, en utilisant alors par mole de composé (III) 1 à 1,5 mole d'alcoolate alcalin.

**2.** Procédé suivant la revendication 1, caractérisé en ce qu'on opère dans la variante (a) à une température de 20 à 100° C, dans la première étape de la variante (b) à une température de 10 à 35° C et dans la seconde étape de la variante (b) à une température de 20 à 100° C.

**3.** Composés de formule générale

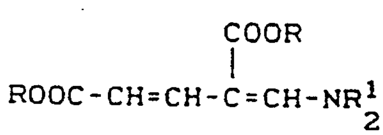

(II)

dans laquelle
R est un reste alkyle en $C_1$ à $C_6$ et
$R^1$ est un reste alkyle en $C_1$ à $C_6$.

**4.** Procédé de préparation de composés de formule

$$ROOC-CH=CH-\overset{\overset{COOR}{|}}{C}=CH-NR^1_2 \qquad (II)$$

dans laquelle
R est un reste alkyle en $C_1$ à $C_6$ et
$R^1$ est un reste alkyle en $C_1$ à $C_6$,
caractérisé en ce qu'on fait réagir des esters d'acide glutaconique de formule (IV)

$ROOC-CH=CH-CH_2-COOR$ (IV)

dans laquelle
R a la définition indiquée ci-dessus, avec des N,N-dialkylformamide-acétals de formule (V) .

$(R^1)_2N-CH(OR^2)_2$ (V)

dans laquelle
$R^1$ a la définition indiquée ci-dessus et
$R^2$ est un groupe alkyle en $C_1$ à $C_6$,
éventuellement en présence d'anhydrides d'acides et, le cas échéant, en présence de diluants inertes, entre 0 et 40 °C.

**5.** Composés de formule (III)

$$ROOC-CH=CH-\overset{\overset{COOR}{|}}{C}=CH-NH_2 \qquad (III)$$

dans laquelle
R est un groupe alkyle en $C_1$ à $C_6$.

**6.** Procédé de préparation de composés de formule (III)

$$ROOC-CH=CH-\overset{\overset{COOR}{|}}{C}=CH-NH_2 \qquad (III)$$

dans laquelle
R est un groupe alkyle en $C_1$ à $C_6$,
caractérisé en ce qu'on fait réagir des esters d'acides 1-dialkylamino-1,3-butadiène-2,4-dicarboxyliques de formule (II)

$$ROOC-CH=CH-\overset{\displaystyle COOR}{\overset{|}{C}}=CH-NR^{1}_{2} \qquad (II)$$

dans laquelle

R a la définition indiquée ci-dessus et

$R^1$ est un groupe alkyle en $C_1$ à $C_6$,

avec l'ammoniac gazeux et en présence de diluants à des températures comprises entre 0° C et 50° C.